# EUROPÄISCHE PATENTANMELDUNG

(11) **EP 2 854 109 A1**
(43) Veröffentlichungstag der Anmeldung: **01.04.2015**
(21) Anmeldenummer: 14185917.3
(22) Anmeldetag: 23.09.2014
(51) Int. Cl.: G06T 17/00, A61F 2/28

(54) **Verfahren zur Nachbildung von Knochen und Planung eines operativen Eingriffs**

(30) Priorität: 27.09.2013 DE 102013110699
(71) Anmelder: Prückmaier, Ludwig, 85560 Ebersberg (DE); Pende, Helmut Hans Boris, 83539 Pfaffing (DE)
(72) Erfinder: Prücklmaier, Ludwig, 85560 Ebersberg (DE)
(74) Vertreter: Weickmann & Weickmann

(57) **Zusammenfassung**

Die Erfindung betrifft ein Verfahren zur Nachbildung von Knochen und Planung eines operativen Eingriffs. Dieses weist folgende Schritte auf:
• Bereitstellen eines digitalen Datensatzes einer 3D-Aufnahme der Knochen mittels digitalem Röntgen, Orthopantomographie (OPT), Computertomographie (CT), digitaler Volumentomographie (DVT) und/oder Magnetresonanztomographie (MRT);
• Identifizierung von Abweichungen von der regelhaften Anatomie anhand der 3D-Aufnahme, insbesondere unfallbedingte, anatomische Veränderungen;
• Erstellen eines realen 3D-Modells, das der 3D-Aufnahme entspricht; und
• Identifizierung der Abweichungen anhand des 3D-Modells zur Planung eines operativen Eingriffs, wobei
- das reale 3D-Modell die Anatomie der Knochen einschließlich der Abweichungen exakt wiedergibt und
- bei der Planung die Anordnung der Knochen und von Knochenbruchstücken untersucht wird sowie die Zugriffsmöglichkeit auf die Knochenbruchstücke und deren Entfernbarkeit festgestellt wird.

## Beschreibung

### I. Anwendungsgebiet

Die vorliegende Erfindung behandelt ein Verfahren zur Nachbildung von Knochen oder Weichgewebe und zur Planung eines operativen Eingriffs. Insbesondere behandelt die Erfindung dabei eine Eingriffssimulation an einem nachgebildeten Schädel. Dabei können Abweichungen von der regelhaften Anatomie festgestellt werden.

Unter der Anatomie werden Gestalt, Lage und Struktur von Körperteilen, Organen, Geweben, Zellen als auch des Skeletts betrachtet. Da beispielsweise bei Knochen die Gestalt, Lage und die Größenverhältnisse der Knochen nur relativ geringfügige Toleranzen erlauben, kann von einer regelhaften Anatomie bei einem gesunden Menschen gesprochen werden. Von dieser regelhaften Anatomie können jedoch Abweichungen auftreten. Dabei ist zunächst zwischen angeborenen, anatomischen Veränderungen und erworbenen, anatomischen Veränderungen zu unterscheiden. Bei den erworbenen anatomischen Veränderungen kann es sich beispielsweise um krankheitsbedingte oder um unfallbedingte Veränderungen handeln. In jedem Fall kann ein operativer, korrigierender Eingriff sinnvoll sein, insbesondere bei unfallbedingten, anatomischen Veränderungen ist der Eingriff zudem äußerst dringlich.

Gerade bei einer schwerwiegenden Kopfverletzung ist es jedoch sinnvoll, vor einem Eingriff den aktuellen Istzustand zu überprüfen, um somit eine entsprechende Operationsstrategie vorbereiten zu können. Hierfür bestehen unterschiedliche Aufnahmeverfahren, wie beispielsweise digitale Röntgenaufnahmen, Computertomographie (CT), Magnetresonanztomographie (MRT), digitale Volumentomographie (DVT) usw.

Selbst wenn sich jedoch der operierende Chirurg mittels solcher Aufnahmeverfahren vorab ein Bild von dem Istzustand hat verschaffen können, hat er trotzdem bei einer schweren Verletzung, wie beispielsweise einem Schädelbasisbruch, große Schwierigkeiten, die Operation wie geplant durchzuführen. Dies liegt zum einen daran, dass bei der tatsächlichen Operation größere Mengen Blut austreten können, die die Übersichtlichkeit der zerbrochenen Schädelteile erschweren. Andererseits bilden die Aufnahmen, die an einem Computerdisplay dargestellt werden, den realen Istzustand der Verletzungen nur unbefriedigend ab, da bei der angezeigten 2D-Aufnahme am Computerdisplay Größenverhältnisse einzelner Bestandteile falsch interpretiert werden können. Dabei ist auch zu beachten, dass es sich bei dem operierenden Chirurgen um keinen Theoretiker handelt. Seine Arbeit und Spezialisierung liegt vielmehr in der praktischen, handwerklichen Durchführung von Operationen. Daher erscheint für ihn eine Visualisierung mittels Computerdisplays oftmals als unbefriedigend.

### II. Technischer Hintergrund

Aus dem Stand der Technik ist es daher bekannt, anatomische Modelle in der Human- und Veterinärmedizin herzustellen, anhand derer ein operierender Arzt eine Operationsstrategie planen kann.

Aus DE 102007042922A1 ist beispielsweise ein Verfahren zum Herstellen anatomischer Modelle in der Human- und Veterinärmedizin bekannt, bei der das Modell mittels der Rapid-Prototyping-Technik hergestellt wird. Unter Rapid-Prototyping-Technologie ist beispielsweise 3D-Druck oder die Stereolithographie zur Erzeugung eines greifbaren anatomischen 3D-Modells zu verstehen. Zur Bildakquisition der Patientendaten wird 2D- oder 3D-Ultraschall mit einem Ultraschallgerät vorgeschlagen, da CT-Aufnahmen eine zu hohe Strahlenbelastung für den Patienten bedeuten würden und MRT-Aufnahmen zu kostenintensiv sind. Mittels des Ultraschallgeräts können Organe, wie beispielsweise Leber oder Herz, plastisch aufgenommen werden, um somit Krankheitsbilder zu identifizieren. Dabei werden die Bilddaten des Ultraschallgeräts in eine Bildverarbeitungssoftware importiert und eine Segmentierung der relevanten anatomischen Strukturen, das heißt der interessierenden Organe, vorgenommen. Basierend auf den Daten wird ein reales Modell erstellt, das der Arzt zur Unterstützung in der Diagnostik verwenden kann und ebenfalls eine Operationsstrategie an Hand des Modells planen kann.

Nachteilhaft an dem vorgeschlagenen Verfahren ist, dass es sich nur schlecht auf die Nachbildung von Knochen oder zur Identifizierung von Abweichungen von der regelhaften Anatomie anwenden lässt. Bei Knochenbrüchen, beispielsweise bei einem Schädelbruch, könnte mittels des vorgeschlagenen Verfahrens nur ein relativ ungenaues Bild des tatsächlichen Istzustands wiedergegeben werden.

### III. Darstellung der Erfindung

### a) Technische Aufgabe

Die Erfindung schlägt daher ein Verfahren zur Nachbildung von Knochen und Planung eines operativen Eingriffs vor, bei dem eine erforderliche Genauigkeit der nachgebildeten Knochen erreicht werden kann und dadurch eine substantiierte Operationsstrategie geplant werden kann.

### b) Lösung der Aufgabe

Die Erfindung hat sich die Erkenntnis zu Nutze gemacht, dass 3D-Drucker der aktuellsten Versionen eine bisher nicht dagewesene Auflösung erreichen können. Im Rahmen der vorliegenden Erfindung ist beispielsweise der 3D-Drucker Projet 3510 MP einsetzbar, der von der Firma 3 D Systems hergestellt wird.

Mittels der vorgeschlagenen 3D-Drucker ist ein Auflösungsvermögen von 0,025 - 0,05 mm möglich.

Um eine entsprechend genaue Nachbildung bei den gedruckten 3D-Modellen erzielen zu können, wird erfindungsgemäß weiterhin vorgeschlagen, einen digitalen Datensatz einer 3D-Aufnahme der Knochen mittels digitalem Röntgen, auch Orthopantomographie (OPT), Computertomographie (CT), digitaler Volumentomographie DVT und/oder Magnetresonanztomographie (MRT) bereit zu stellen. Mittels dieser Verfahren weisen nämlich die digitalen 3D-Aufnahmen entsprechend hohe Auflösungen auf.

Im Ergebnis wird so erfindungsgemäß ein reales 3D-Modell des Ist-Zustands des Patienten, d.h. der Anatomie der Knochen einschließlich der Abweichungen von der regelhaften Anatomie, hergestellt, das innerhalb eines Toleranzbereichs von ± 0,05 mm, vorzugsweise ± 0,025 mm im Vergleich zu den tatsächlichen Knochen des Patienten liegt.

Weiterhin wird erfindungsgemäß vorgeschlagen, dass das reale 3D-Modell bei der Planung der Operationsstrategie eingesetzt wird. Dabei werden die Anordnung der Knochen und die Anordnung von Knochenbruchstücken untersucht. Zudem wird die Zugriffsmöglichkeit auf die Knochenbruchstücke und deren Entfernbarkeit anhand des realen 3D-Modells festgestellt.

Aufgrund der erfindungsgemäßen, hohen Abbildungsgenauigkeit der Knochen durch das reale 3D-Modell und der damit verbundenen sehr hohen Zuverlässigkeit des 3D-Modells können anhand des 3D-Modells weitergehende Operationsstrategien geplant werden, wie es beim Stand der Technik unmöglich war. Bei dem erfindungsgemäßen 3D-Modell, bei dem es sich beispielsweise um einen Schädel oder um einen Teilschädel handeln kann, können nämlich auch kleinste Splitter von beispielsweise 2 mm oder kleiner als 1 mm exakt identifiziert werden bezüglich ihrer Größe und Anordnung, sodass es sich anhand des 3D-Modells sofort für den operierenden Chirurgen feststellen lässt, wie bei dem entsprechenden Splitter am besten vorzugehen ist. Dabei besteht eine Problematik darin, wie am besten der Zugriff auf die einzelnen Knochen, Knochenteile oder Knochenbruchstücke erfolgen kann und andererseits darin, wie die einzelnen Knochen bzw. Knochenbruchstücke bei der Operation behandelt werden.

Vorzugsweise wird bei identifizierten Knochenbruchstücken zunächst geprüft, ob diese wiederverwertet werden können. Darunter ist zu verstehen, ob die Knochenbruchstücke wieder in den zerbrochenen Knochen eingesetzt werden können, um somit die regelhafte Anatomie wieder herstellen zu können. Falls entschieden wird, dass die Knochenbruchstücke nicht wieder verwertet werden können, so wird zudem vorzugsweise geprüft, wie die Knochenbruchstücke am besten entfernt werden können. Dabei ist insbesondere zu prüfen, in welcher Orientierung die Knochenbruchstücke am einfachsten und mit dem geringsten zusätzlichen Verletzungsrisiko entfernt werden können.

Es wird zudem vorgeschlagen, dass bei der Nichtwiederverwertbarkeit von Knochenbruchstücken entsprechende Knochenersatzteile auf Basis der 3D-Aufnahme digital konzeptioniert und im Anschluss als physische Teile aus einem geeigneten Material hergestellt werden. Somit besteht bei der erfindungsgemäßen Operationsplanung der Vorteil darin, dass nicht nur die Abweichungen von der regelhaften Anatomie anhand des 3D-Modells korrekt identifiziert werden können, sondern dass bereits abschließend sämtliche Vorbereitungen für die Operation vorgenommen werden können, sodass bei der Operation der Knochen wieder so zusammengebaut werden kann, dass er der regelhaften Anatomie entspricht. Somit dient das erfindungsgemäße Verfahren der Vorbereitung lediglich eines einzigen operativen Eingriffs.

Vorteilhafter Weise erfolgt die Konzeption eines Knochenersatzteiles dadurch, dass, falls mehrere Knochenbruchstücke durch das Knochenersatzteil zu ersetzen sind, die Knochenbruchstücke zusammengesetzt werden, um somit die Form des Knochenersatzteiles zu erhalten. Dies kann einerseits anhand des realen 3D-Modells, insbesondere durch den operierenden Chirurgen, vorgenommen werden. Diese praktische Vorgehensweise hat den Vorteil, dass sich der operierende Chirurg ein Bild des tatsächlich notwendigen Knochenersatzteiles machen kann. Oftmals erhält der Chirurg dadurch zusätzliche Hinweise, die so aus der digitalen Konzeption, die in erster Linie ein Techniker vornimmt, nicht hervorgehen würden. Hauptsächlich soll jedoch die Konzeptionierung der Knochenersatzteile auf Basis der digitalen 3D-Aufnahme erfolgen. Dabei können, ausgehend von der 3D-Aufnahme als digitales Ist-Modell, die zu ersetzenden Knochenbruchstücke identifiziert werden und am Computer zusammengesetzt werden, um somit eine digitale 3D-Aufnahme des zu erzielenden Knochenersatzteiles zu erhalten. Diese 3D-Aufnahme des Knochenersatzteils dient dann vorzugsweise als Basis für die Herstellung des physischen Knochenersatzteils. Dabei können in den Datensatz noch die Erkenntnisse des operierenden Chirurgen einfließen, die er bei der Überprüfung des realen 3D-Modells und der Zusammenfügung der Knochenbruchstücke erlangt hat.

Es wird zudem vorgeschlagen, dass ausgehend von der 3D-Aufnahme als digitales Ist-Modell die zu ersetzenden Knochenbruchstücke herausgefiltert und durch das eine oder die mehreren Knochenersatzteile ersetzt werden. Die Knochenersatzteile können an die vorgesehenen Positionen in den übrigen Knochen eingefügt werden, um ein digitales 3D-Soll-Modell zu erhalten. Dieses 3D-Soll-Modell entspricht dann wieder weitestgehend der regelhaften Anatomie.

Zum Erhalten des digitalen 3D-Soll-Modells können zudem auch die wiederzuverwertenden Knochenbruchstücke an die vorgesehenen Positionen versetzt werden. Bei dem so zumindest theoretisch fertiggestellten digitalen 3D-Soll-Modell kann es jedoch nun passieren, dass der Knochen immer noch nicht vollständig ist, da Spalte, Löcher oder dergleichen bestehen. Dementsprechend wird vorzugsweise noch eine abschließende Überprüfung vorgenommen, bei der in diesem Falle die Knochenersatzteile noch einmal entsprechend korrigierend angepasst werden können.

Das somit endgültig fertiggestellte digitale 3D-Soll-Modell kann, falls vom Chirurgen erwünscht, zusätzlich als reales 3D-Modell, beispielsweise mittels 3D-Druck, hergestellt werden.

Es kann nun der Fall auftreten, dass einzelne Knochenteile oder Knochenbruchstücke im Körper des Patienten "frei schwebend" sind, was bedeutet, dass diese beispielsweise vollständig im Fleisch oder im Gehirn stecken und somit keine Verbindung zu den übrigen Knochen aufweisen. Es wird vorgeschlagen, dass solche frei schwebenden Knochenteile oder Knochenbruchstücke in der 3D-Aufnahme vor Herstellung des realen 3D-Modells identifiziert werden. Es können dann in die digitale 3D-Aufnahme entsprechende Abstandshalter zwischen den frei schwebenden Knochenteilen oder Knochenbruchstücken und den übrigen Knochen eingearbeitet werden. Die Abstandshalter haben die Funktion, die frei schwebenden Knochenteile oder Knochenbruchstücke in dem später hergestellten realen 3D-Modell an ihrer tatsächlichen Position festzuhalten.

### c) Ausführungsbeispiele

Ausführungsformen gemäß der Erfindung sind im Folgenden beispielhaft näher beschrieben. Es zeigen:
- Fig. 1:: eine erste beispielhafte Ausführungsform eines erfindungsgemäßen Verfahrens;
- Fig. 2:: eine zweite Ausführungsform des erfindungsgemäßen Verfahrens.

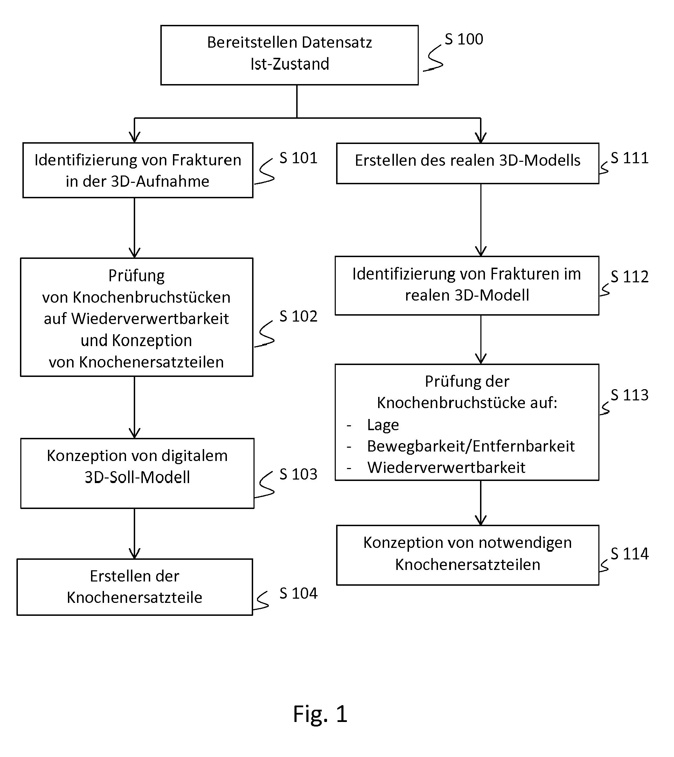

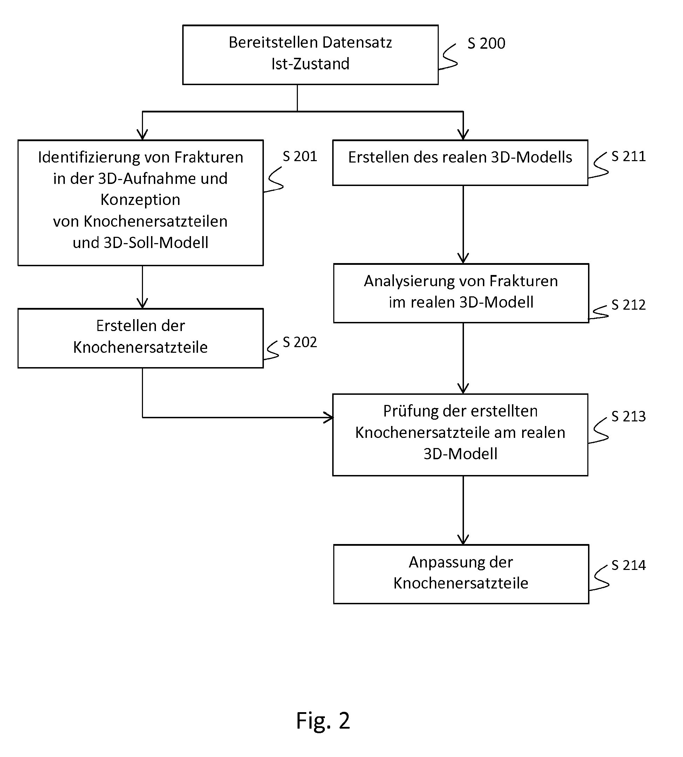

Bei den Ausführungsformen aus Figur 1 und Figur 2 wird beides Mal davon ausgegangen, dass ein Patient mit einer schwerwiegenden Schädelverletzung, wie etwa einem Schädelbasisbruch in ein Krankenhaus zur sofortigen Behandlung eingeliefert wird. In einem solchen Fall ist die Dringlichkeit einer möglichst raschen Operation besonders hoch, das bedeutet, dass die Behandlung in kürzester Zeit mit möglichst wenigen Schritten zu erfolgen hat. Dies wird durch das erfindungsgemäße Verfahren gewährleistet.

In Figur 1 in Schritt S100 wird zunächst ein digitaler Datensatz einer 3D-Aufnahme des Schädels des Patienten bereitgestellt. Die Bereitstellung erfolgt nach Durchführung der üblichen, oben genannten Verfahren, wie beispielsweise mittels Computertomographie. Üblicher Weise wird bei den erfassten Rohdaten noch eine Segmentierung, Filterung etc. vorgenommen, wie dies aus dem Stand der Technik bekannt ist, um die relevanten Körperteile, in diesem Fall das Skelett bzw. die Knochen, herauszufiltern bzw. besser sichtbar zu machen.

Der Patient wird üblicherweise bis zur Operation in ein künstliches Koma versetzt, um seine Überlebenschancen zu erhöhen.

Ausgehend von den in Schritt S100 bereitgestellten Daten erfolgen nun zwei parallele Abläufe, nämlich die Verarbeitung der digitalen 3D-Aufnahme in den Schritten S101 bis S103, sowie die Erstellung eines realen, physischen 3D-Modells und eine Analyse anhand des realen 3D-Modells in den Schritten S111 bis S114. Eine solche Vorgehensweise hat einerseits den Vorteil, dass durch das parallele Vorgehen Zeit eingespart werden kann und andererseits zwei voneinander losgelöste, unabhängige Analysen des Ist-Zustands des Patienten, das heißt seiner Verletzungen, vorgenommen wird, sodass die Zuverlässigkeit der Analyse und somit der Operationsvorbereitungen deutlich erhöht werden kann. Die Erfolgschancen der Operation können somit ebenfalls erhöht werden.

Im Schritt S101 werden anhand der digitalen 3D-Aufnahme, das heißt an einem Computer, die am Schädel bestehenden Frakturen, das heißt Brüche, identifiziert. Daraufhin wird analysiert, wie die einzelnen Knochenbruchstücke ausgebildet sind. Es wird in Schritt 102 geprüft, welche der Knochenbruchstücke wieder verwertet werden können, das heißt, wieder in den Schädel eingesetzt werden können. Für diejenigen Knochenbruchstücke, die nicht wieder verwertet werden können, werden digital Knochenersatzteile konzeptioniert. Dies kann beispielsweise dadurch erfolgen, dass die durch ein Knochenersatzteil zu ersetzenden Knochenbruchstücke herausgefiltert und digital zusammengesetzt werden.

Ausgehend von den am Computer gemachten Analysen kann nunmehr ein digitales 3D-Soll-Modell in Schritt S103 konzeptioniert werden. Bei diesem wurden alle wieder zu verwendenden Knochenbruchstücke sowie die konzeptionierten Knochenersatzteile an die vorhergesehenen Lagen im Schädel verschoben. Dadurch kann allein aufgrund der digitalen Daten eine Überprüfung der in den Schritten S101 und S102 vorgenommenen Arbeitsschritte vorgenommen werden.

Parallel zu den sich einzig mit den digitalen Daten beschäftigenden Arbeitsschritten S101 bis S103 wird in Schritt S111 ein reales, physisch vorhandenes 3D-Modell erstellt, insbesondere mittels eines 3D-Druckers. Dieser Vorgang dauert vorzugsweise nicht länger als 8 oder 12 Stunden. Da dieser Vorgang jedoch immer noch relativ zeitaufwendig ist, sollte er unmittelbar nach der Bereitstellung des Datensatzes in Schritt S100 begonnen werden.

Sobald das reale 3D-Modell des Schädels fertig gestellt worden ist, können an diesem die bestehenden Frakturen durch die operierenden Ärzte, insbesondere durch die oder den Chirurgen, in Schritt S112 untersucht werden.

Die Ärzte prüfen zunächst in Schritt S113, wie die bestehenden Knochenbruchstücke ausgebildet sind. Dies bedeutet, dass sie prüfen, welche Form und Größe die Knochenbruchstücke aufweisen und wo sie angeordnet sind. Daraus können sich bereits wichtige Schlussfolgerungen ergeben, beispielweise wenn ein Knochenbruchstück ins Hirn eingedrungen ist bzw. das Hirn verletzt hat. In diesem Fall ist es nämlich möglicherweise notwendig, dass für die Operation zusätzliche Maßnahmen bezüglich der Behandlung des Gehirns vorbereitet werden.

Zudem kann der Chirurg in Schritt S113 auch auf rein handwerkliche Weise feststellen, welche Bewegbarkeit ein bestimmtes Knochenbruchstück aufweist, das heißt, ob es beispielsweise verkeilt ist, ob es sich drehen lässt oder ob es von außen zugänglich ist. Dabei interessiert vor allem, ob sich das Knochenbruchstück mittels geeigneter Instrumente von außen durch eine Öffnung herausziehen lässt, ohne dabei weitere Verletzungen am Schädel zu verursachen. Aufgrund der Tatsache, dass der erstellte Schädel als physischer Körper vor dem behandelnden Chirurgen liegt, kann dieser aufgrund seiner Erfahrung zumeist mit einem Blick ein zutreffendes Urteil fällen.

Weiterhin werden im Schritt S113 die Knochenbruchstücke auf Wiederverwendbarkeit geprüft. Bei größeren Stücken, wenn beispielsweise ein Teil des Schädels lediglich angebrochen ist, kann eine korrigierende Neuausrichtung des Knochenbruchstücks die sinnvollste Lösung sein. Bei kleineren, insbesondere splitterartigen Knochenbruchstücken ist jedoch zumeist ein Knochenersatzteil, das mehrere, kleinere zersplitterte Knochenbruchstücke ersetzt, notwendig. Oftmals kann der behandelnde Arzt allein durch Betrachtung des rea-len 3D-Modells die Notwendigkeit von Knochenersatzteilen feststellen.

Für eine Konzeption der notwendigen Knochenersatzteile in Schritt S114 ist es jedoch oftmals auch sinnvoll, die zu ersetzenden Knochenbruchstücke aus dem 3D-Modell zu entfernen und - soweit möglich - puzzleartig zusammen zu setzen. Die Entfernung der Knochenbruchstücke aus dem 3D-Modell mittels geeigneter Operationsgeräte kann dabei zusätzlich als Training für die spätere, tatsächliche Operation am Patienten dienen. Insbesondere können dabei noch einmal die Größenverhältnisse der Knochenbruchstücke zu dem gebrochenen Schädel überprüft werden, insbesondere, ob die Knochenbruchstücke tatsächlich wie angenommen sich aus Öffnungen im Schädel entnehmen lassen.

Sobald die operierenden Ärzte auf ihrer Seite die notwendigen Knochenersatzteile konzeptioniert haben bzw. ihre Schlussfolgerungen bezüglich der Eigenschaften und Notwendigkeit von Knochenbruchstücken gemacht haben, können Sie diese Informationen in das im Schritt S103 konzeptionierte, digitale 3D-Soll-Modell einfließen lassen. Da das in Schritt S103 digital konzeptionierte 3D-Soll-Modell in erster Linie von Technikern und/oder technisch versierten Medizinern konzeptioniert worden ist, kann somit in diesem Schritt in einer entsprechenden Diskussion ein Abgleich zwischen der digitalen Konzeption und der am realen 3D-Modell handwerklich geprägten Konzeption erfolgen.

Basierend auf den in Schritt S103 gemachten Erkenntnissen bezüglich des zu erzielenden 3D-Soll-Modells werden anschließend im Schritt S104 die notwendigen Knochenersatzteile erstellt. Dies kann beispielsweise mittels Fräsen oder Sintern erfolgen. Als Material für die Knochenersatzteile kann Zirkonium, Titan, Kunststoff oder Stahl in Frage kommen. Eine alternative Möglichkeit kann darin bestehen, dass die Knochenersatzteile mittels 3D-Druck hergestellt werden. Damit das Druckmaterial auch geeignet ist und vor allem vom Körper angenommen wird, könnten hierfür beispielsweise patienteneigene Zellkulturen in Frage kommen. Aufgrund der Dringlichkeit sollte die Herstellung der Knochenersatzteile nicht mehr als 12 oder 24 Stunden in Anspruch nehmen, am besten weniger als 8 Stunden.

Abschließend besteht noch theoretisch die Möglichkeit, dass die fertig gestellten, realen Knochenersatzteile am realen 3D-Modell anprobiert und gegebenenfalls zusätzlich angepasst werden.

Figur 2 zeigt eine zu Figur 1 alternative Ausführungsform, die jedoch alle Schritte der Ausführungsform von Figur 1 aufweist, jedoch in einem anderen Ablauf. Der wesentliche Unterschied besteht darin, dass die Erstellung des realen 3D-Modells und die Erstellung der Knochenersatzteile parallel ablaufen. Da diese beiden Arbeitsschritte am zeitaufwendigsten sind, hat diese Vorgehensweise den Vorteil, dass sie insgesamt weniger Zeit in Anspruch nimmt.

In Schritt S200 wird erneut der digitale Datensatz des Ist-Zustandes des Patienten bereitgestellt.

Darauf basierend wird in Schritt S201 eine Identifizierung der Frakturen vorgenommen. Dabei wird außerdem geprüft, welche Knochenbruchstücke bestehen und welche davon wiederverwertbar sind. Basierend auf diesen Analysen werden ebenfalls wie in der Ausführungsform von Figur 1 digital die notwendigen Knochenersatzteile konzeptioniert. Ebenfalls wird wie in der Ausführungsform von Figur 1 ein digitales 3D-Soll-Modell konzeptioniert.

Anschließend werden die in den Patienten bei der Operation einzusetzenden Knochenersatzteile in Schritt S202 erstellt. Der Unterschied zur Ausführungsform von Figur 1 besteht also darin, dass die Knochenersatzteile allein auf Basis der digitalen Daten erstellt werden, ohne dass die Erkenntnisse der Analysen des realen 3D-Modells mit einfließen.

Dies liegt daran, dass bei der Ausführungsform von Figur 2 das reale 3D-Modell zum Zeitpunkt des Beginns der Erstellung der Knochenersatzteile im Schritt S202 noch gar nicht fertig gestellt worden ist. Ausgehend von dem in Schritt 200 bereitgestellten Datensatz des Ist-Zustandes des Schädels des Patienten wird in Schritt S211 das reale 3D-Modell des Schädels hergestellt, beispielsweise gedruckt.

In Schritt S212 werden am fertig gestellten 3D-Modell des Schädels in gleicher Weise wie in der Ausführungsform von Figur 1 (in den Schritten S112, S113, S114) die Frakturen am Schädel analysiert.

Sobald die Erstellung der realen Knochenersatzteile in Schritt S202 abgeschlossen ist, wird in Schritt S213 geprüft, ob die erstellten Knochenersatzteile in das reale 3D-Modell des Schädels wie vorgesehen hineinpassen. Es erfolgt also eine Einprobe der erstellten Knochenersatzteile am Schädelmodell.

Dabei kann es sich ergeben, dass in Schritt S214 zusätzliche Anpassungen der erstellten Knochenersatzteile notwendig sind. Dabei ist es auch denkbar, dass die Knochenersatzteile insbesondere an ihren Kontaktflächen absichtlich geringfügig zu groß hergestellt worden sind, sodass anschließend bei der Einprobe am Schädelmodell eine Anpassung durch nachträgliches Schleifen, insbesondere der Kontaktflächen, erfolgen kann.

## Patentansprüche

1. Verfahren zur Nachbildung von Knochen und Planung eines operativen Eingriffs, aufweisend die folgenden Schritte:
Bereitstellen eines digitalen Datensatzes einer 3D-Aufnahme der Knochen mit-tels digitalem Röntgen, Orthopantomographie (OPT), Computertomographie (CT), digitaler Volumentomographie (DVT) und/oder Magnetresonanztomographie (MRT);
Identifizierung von Abweichungen von der regelhaften Anatomie anhand der 3D-Aufnahme, insbesondere unfallbedingte, anatomische Veränderungen;
Erstellung eines realen 3D-Modells, das der 3D-Aufnahme entspricht;
Identifizierung der Abweichungen anhand des realen 3D-Modells zur Planung eines operativen Eingriffs, wobei
das reale 3D-Modell die Anatomie der Knochen einschließlich der Abweichungen innerhalb eines Toleranzbereichs von ±0,05 mm exakt wiedergibt, und
bei der Planung die Anordnung der Knochen und von Knochenbruchstücken untersucht wird sowie die Zugriffsmöglichkeit auf die Knochenbruchstücke und deren Entfernbarkeit festgestellt wird.

2. Verfahren nach Anspruch 1,
wobei die Knochenbruchstücke auf Wiederverwertbarkeit überprüft werden und bei Nichtwiederverwertbarkeit entsprechende Knochenersatzeile auf Basis der 3D-Aufnahme konzeptioniert und hergestellt werden.

3. Verfahren nach Anspruch 2,
wobei bei der Konzeption eines Knochenersatzteiles die zu ersetzenden Knochenbruchstücke identifiziert werden und das Knochenersatzteil durch Zusammensetzen der Knochenbruchstücke erstellt wird.

4. Verfahren nach einem der vorherigen Ansprüche,
wobei ausgehend von der 3D-Aufnahme als digitales Ist-Modell die zu ersetzenden Knochenbruchstücke herausgefiltert und durch Knochenersatzeile ersetzt werden, und
die Knochenersatzeile an die vorgesehenen Positionen in den übrigen Knochen eingefügt werden, um ein digitales 3D-Soll-Modell zu erhalten.

5. Verfahren nach Anspruch 4,
wobei zum Erhalten des digitalen 3D -Soll-Modells auch die wiederverwerteten Knochenbruchstücke an die vorgesehen Positionen versetzt werden.

6. Verfahren nach einem der vorherigen Ansprüche 4 oder 5,
wobei eine abschließende Überprüfung des fertiggestellten digitalen 3D-Soll-Modells erfolgt, bei der die Knochenersatzteile, falls notwendig, an die übrigen Knochen und die wiederverwerteten Knochenbruchstücke angepasst werden.

7. Verfahren nach einem der vorherigen Ansprüche 2-6,
wobei die Konzeption der Knochenersatzteile zunächst anhand des realen 3D-Modells erfolgt.

8. Verfahren nach einem der vorherigen Ansprüche,
wobei das reale 3D-Modell mittels 3D-Druck hergestellt wird.

9. Verfahren nach einem der vorherigen Ansprüche,
wobei das reale 3D-Modell die Anatomie der Knochen einschließlich der Abweichungen innerhalb eines Toleranzbereichs von ±0,025 mm exakt wiedergibt.

10. Verfahren nach einem der vorherigen Ansprüche,
wobei freischwebende Knochenbruchstücke, die keine Verbindung zu den übrigen Knochen aufweisen, in der 3D-Aufnahme vor Herstellung des realen 3D-Modells identifiziert werden, und
in die 3D-Aufnahme entsprechende Abstandshalter zwischen den freischwebenden Knochenbruchstücken und den übrigen Knochen eingearbeitet werden, um die freischwebenden Knochenbruchstücke zu halten.

11. Verfahren nach einem der vorherigen Ansprüche,
wobei die Knochenersatzteile aus Zirkonium, Titan, Kunststoff oder Stahl gefräst und/oder gesintert werden.

12. Verfahren nach einem der vorherigen Ansprüche,
wobei die Knochenersatzteile mittels 3D-Druck hergestellt werden.

13. Verfahren nach Anspruch 12,
wobei Zellkulturen des Patienten als 3D-Druckmaterial dienen.

14. Verfahren nach einem der vorherigen Ansprüche 2-13,
wobei die Herstellung des realen 3D-Modells und der Knochenersatzeile gleichzeitig erfolgt.

15. Verfahren nach einem der vorherigen Ansprüche 2-14,
wobei das reale 3D-Modell innerhalb von weniger als 12 Stunden hergestellt wird, und/oder
die Knochenersatzteile innerhalb von weniger als 12 oder 24 Stunden hergestellt werden.
